# EUROPEAN PATENT SPECIFICATION

(11) **EP 0 467 991 B1**
(45) Date of publication and mention of the grant of the patent: **12.08.1998**
(21) Application number: 90908046.7
(22) Date of filing: 12.04.1990
(51) Int. Cl.: A61K 35/14, C12N 7/04, C12N 7/06

(54) **PLATELET MEMBRANE MICROPARTICLES**
BLUTPLÄTTCHENMEMBRAN-MIKROTEILCHEN
MICROPARTICULES DE MEMBRANES DE PLAQUETTES SANGUINES

(30) Priority: 14.04.1989 US 337916
(43) Date of publication of application: 29.01.1992
(60) Divisional application: 97113637.9
(73) Proprietor: PRP, INC., West Newton, MA 02165 (US)
(72) Inventor: FRANCIS, Chao, Newton, MA 02158 (US)
(74) Representative: Jump, Timothy John Simon
(86) International application number: PCT/US90/01989
(87) International publication number: WO 90/12581

(56) References cited:
- US-A- 4 540 573
- US-A- 4 760 131
- Biological Abstracts, Volume 82, No. 8 Issued 1986, (GEORGE et al), "Platelet Membrane Microparticles in blood bank fresh frozen plasma and cryoprecipitate". See entire document.

## Description

This invention relates generally to the field of medicine and more particularly to a platelet membrane microparticle preparation for use in transfusions to control bleeding.

The body of a normal adult contains 4.0 to 5.5 liters of blood, composed of approximately 60% fluid (plasma) and 40% formed elements (red cells; white cells; and platelets). Under normal physiological conditions, the main function of the platelets is prevention of hemorrhage (bleeding).

Platelets are formed in the bone marrow from precursor cells called megakaryocytes and have a life span of 8 to 10 days in circulation. As a consequence of this short life span, platelet deficiency occurs rapidly when the ability of the bone marrow to produce platelets is depressed, such as in cancer patients undergoing chemotherapy. Platelet deficiency also may occur as a result of various diseases, for example when antibodies are produced in vivo against platelet surface glycoproteins or against other platelet surface antigens. Such depletion or destruction of platelets results in an insufficient quantity of circulating platelets (a condition referred to as thrombocytopenia) and can cause uncontrolled bleeding. Platelet deficiency also may result from surgery involving for example extracorporeal circulation which tends to damage or destroy circulating platelets. Clinical management of thrombocytopenia typically has involved transfusion of fresh, intact platelets.

It is predominantly held that only metabolically-active, intact platelets can function in vivo to arrest bleeding. As a consequence, transfusion therapy has been dependent on the procurement of high quality, fresh, viable platelets. Platelets for transfusion typically are prepared: (a) from freshly donated blood units, called random-donor platelets or (b) from a single donor by apheresis, called single-donor platelets. These transfusion products are plasma suspensions of fresh, concentrated, intact platelets.

A major drawback of the current practice of platelet transfusion is the short shelf life of intact platelets, three to five days. Many platelet units collected by hospitals, bloodbanks and the like unfortunately are discarded due to outdating. Because of their short shelf life, it is very difficult to maintain large inventories of platelet units. This problem is particularly critical in connection with decentralized operations, such as the military, civil defense and disaster agencies.

Several substitutes for intact, viable platelets have been attempted for transfusion both clinically and in animal models. In 1956 (1), it was reported that hemostasis was achieved following the clinical administration of lyophilized platelets, suggesting that the morphological integrity of platelets may not be essential for the retention of at least some of the in vivo functions of intact platelets. A major side effect of the intravenous administration of the lyophilized platelet material was that the patient experienced severe pain at the site of infusion, possibly due to vasospasm caused by the high serotonin content in the lyophilized material. Contrary to the foregoing result, it was reported in 1959 (2) that fresh, ultrasound-disrupted whole platelet preparations failed to reduce the number of erythrocytes in the lymph of thrombocytopenic dogs. More recently, McGill et al (3) reported that the transfusion of platelet membrane concentrates shortened bleeding times in thrombocytopenic rabbits. The concentrate included ghost platelets about the size of a normal platelet and containing mitochondria and remnants of the surface-connecting system. McGill's concentrate was prepared by: (1) centrifuging the whole blood of rabbits to pellet fresh platelets; (2) freezing the pellet at -65°C; (3) thawing and then twice-freezing and thawing the pellet; and (4) rinsing and resuspending the pellet in platelet-free plasma.

In preparing the foregoing platelet membrane fractions, temperature conditions of about 4°C. or below were maintained. Such temperatures are standard when working with biologicals as activity is routinely lost in very short intervals when biologicals are exposed to higher temperatures. For example, proteins such as enzymes may be inactivated by heating them to about 60°C. Activity may be lost even at 4°C. For example, it has been reported that partially purified platelet factor 3 (PF-3) loses a major portion of its clotting activity after five days storage at 4°C (4). (PF-3 appears to be associated with a platelet membrane complex that provides a catalytic surface to promote thrombin generation.) Such loss of activity is of great concern when considering the use of a platelet fraction as a pharmaceutical.

George et al (6) reported that both fresh frozen plasma and cryoprecipitate (the latter having been prepared by separating plasma from blood, freezing the plasma at -65°C, thawing it to 4°C, centrifuging the thawed plasma, expressing off the supernatant plasma and refreezing the remaining plasma and precipitate at -65°C) contain a significant quantity of platelet membrane microparticles, the microparticles being more concentrated in the cryoprecipitate. George speculated that the platelet membrane microparticles present in cryoprecipitate could have a haemostatic function, possibly as a result of the dispersion of the platelets' contact-promoting membrane glycoproteins on small particles.

When preparing a platelet fraction for use in humans, it is of course necessary to use sterile conditions. Like whole blood transfusions, the use of donated platelet units exposes the recipients to the risk of transmission of diseases such as AIDS, hepatitis, and other transfusion-related diseases. Another risk is that after multiple transfusions, the recipient/patients may develop antibodies against donated platelets (a condition known as alloimmunization). Such antibodies can cause rapid destruction of the platelets transfused. Further, bacterial contamination of stored platelets is a significant hazard in transfusions.

The invention provides pharmaceutical and diagnostic products derived according to novel methods from platelet membranes. The products include platelet membrane fragment preparations that have procoagulant activity and that may be used as transfusion substitutes for whole platelets to control bleeding, may be used topically to promote wound healing or may be used as a diagnostic agent in vitro or in in vivo.

According to a first aspect of the invention there is provided a method for preparing a platelet derived product having procoagulant activity, characterised by heat-treating a preparation of isolated procoagulantly active platelets or platelet membrane fragments sufficiently thereby to inactivate any virus contained in said preparation.

In a second aspect, the invention provides a platelet membrane fragment preparation having procoagulant activity, characterised by comprising active virus free isolated platelet membrane fragments obtainable by using a method in accordance with the first aspect of the invention. The preferred method for making this preparation involves heat-treating a platelet membrane fragment preparation to reduce or eliminate viral contamination, as well as bacterial contamination. Although the preparation is heat-treated, surprisingly, it retains its procoagulant and hemostatic properties. Moreover, the heat-treated membrane fragment is far more stable than expected. While such treatment is known to inactivate viral contaminants generally, it never has been used in connection with a platelet preparation. The invention therefore provides for the first time a viral-inactivated, platelet fraction useful for transfusion. The preparation is capable of being stored in solution at 4°C for at least eight weeks without significant loss of procoaggulant activity for at least six months (greater than 90% retained). It retains this activity (greater than 90%) even after lyophilization. In addition the protein and phospholipid content do not change after lyophilization and storage for six months.

Preferably, the preparation is nonimmunogenic, and in particular is nonantigenic with respect to class I HLA antigenic determinants. Thus, the suitability of the preparation for a particular recipient is not limited by the genetic characteristics of the donor.

According to yet another aspect of the invention, the platelet membrane fragment preparation, which has procoagulant activity and is capable of controlling bleeding, is substantially free of GP IIb/IIIa complex. This is surprising in view of the widely held belief that GP IIb/IIIa complex is involved in and necessary for hemostasis.

The platelet membrane fragment preparation preferably is substantially free of ghost platelets, and contains relatively homogeneous microparticles. Preferably, at least 80% of the microparticles have a diameter less than 600 nanometers, and at least 95% have a diameter less than one micron. Most preferably, the microvesicles have an average diameter of between about 300 and 400 nanometers. Such microparticles are about 1/5 to 1/7 the size of a typical ghost platelet.

The preferred preparation contains virtually no serotonin (less than .02% of that found in platelet lysates), thereby eliminating the respiratory and vascular problems characteristic when certain of the preparations of the prior art are used for transfusion. The preparation further has no detectable purine nucleoside phosphorylase activity, a cytoplasmic enzyme marker, and is substantially free of factors V, VIII, IX and X. In one embodiment the microparticle fraction has 3% carbohydrate, 30% phospholipid, 58% protein and 9% cholesterol by weight and the ratio of protein versus phospholipid is 1.97 ±0.10 (mean ±SD, n=7).
In preferred embodiments of the inventive method the preparation is homogenized, preferably by sonocation, to form platelet membrane microparticles substantially free of ghost platelets. Homogenization (sonication) which, preferably, follows the heat-inactivation step results both in creating microparticles of homogenous size and in preventing a substantial amount of the activity from being bound up in any precipitate formed during the heat-inactivation step. The preparation also may be treated to remove substantially all GP IIb/III a, a surface glycoprotein complex.

The invention also can involve the use of outdated platelets or derivatives thereof as the starting material. The outdated platelets may be from a single donor source, or may be pooled from multiple sources. There is an increased risk of viral infection with outdated platelets and the invention makes outdated platelets useful by ensuring that they are free or active virus.

By outdated platelets it is meant platelets that have been stored at a temperature of between about 4°C and room temperature for at least three days. Outdated platelets are those which according to accepted practice are discarded as being no longer suitable for use as a transfusion material. Derivatives of platelets means non-intact platelets such as ghost platelets or platelet membrane fragments including platelet membrane microvesicles.

Thus, the invention has as an advantage the provision of a platelet membrane fraction for transfusion prepared wholly or in part from outdated platelets, thereby making use of huge quantities of platelets heretofore discarded as useless.

The most preferred method for preparing the products of the invention involves repeated freeze-thawing and washing platelets to yield primarily ghost platelets and a lysate. The ghost platelets then are separated from the lysate and are suspended in a solution to form a suspension. Then the suspension containing the ghost platelets is heated to at least 60°C for at least two hours to inactivate viral contaminants. The heat treatment also causes a precipitate to form. However, the precipitate is not removed at this point because it contains a significant amount of the desired activity. Instead, the suspension including the precipitate first is homogenized, preferably by sonication, and then the precipitate is separated from the suspension. The suspension then may be stored or used for transfusion.

The platelet membrane fragment preparation may be used in pharmaceutically effective amounts in the treatment of animals or humans to prevent bleeding. When used as a pharmaceutical preparation for transfusions, the sterile preparation may be suspended in any physiologically compatible solution such as saline or plasma. It may be used alone, or with other agents, including whole platelets. The preparation is an ideal additive to artificial blood. The preparation also may be applied topically to stop bleeding and to treat wounds. In this regard the preparation may be suspended in a pharmaceutically acceptable carrier such as a gel or ointment or may be impregnated in a carrier such as gauze. The preparation also may be used as a carrier for drug delivery, or may be labeled and used diagnostically, for example, as an imaging agent to trace the location of a clot.

The products of the invention are administered to subjects in effective amounts. The term "subject" is intended to include living organisms having a hemostatic capability mediated at least in part by platelets, e.g. humans, dogs, cats, horses and the like. An "effective amount" is that amount capable of achieving the particular therapeutic or diagnostic purpose for which the product is administered. In the general case of transfusion, an effective amount is that which will act to restore the hemostatic function to substantially the same level that would be achieved if whole platelets were transfused. In the case of a subject suffering from thrombocytopenia, an effective amount is that amount which is capable of reducing the bleeding time in the subject, preferably to non-dangerous levels and most preferably to levels consistent with normal individuals. An effective amount can be determined on an individual basis and will be based, at least in part, upon the subject's size, the severity of the symptoms to be treated and the results sought. Thus, an effective amount can be determined by one of ordinary skill in the art employing such factors and using no more than routine experimentation.

The products of the invention adventageously are prepared from pooled platelets, which ordinarily would induce an antibody response when transfused into most individuals. Currently, alloimmunization is a difficult problem in the management of thrombocytopenic patients who require repeated platelet transfusion. Platelets exhibit HLA-A and HLA-B antigens which either are a part of platelet membranes or are absorbed from plasma. The development of antibodies against HLA-A or HLA-B (which is the primary cause of platelet alloimmunization) causes rapid destruction of transfused platelets, thus diminishing the effect of transfusion of platelets on hemostasis. Contamination by white cells (which are a ready source of HLA antigens) in platelet concentrates also may contribute to the development of alloimmunization.

However, these problems are solved by preparations of the invention which are nonimmunogenic, nonalloimmunogenic and nonimmunogenic to HLA determinants (particularly Class I HLA determinants). Nonimmunogenic herein means that a subject, after several transfusions, does not mount an immunological response sufficient to interfere with the therapeutic effect of the transfused product. Nonalloimmunogenic herein means that a subject, after several transfusions of material prepared from alloimmunogenic sources, does not exhibit a detectable immunological response against alloantigens. By detectable, it is meant that serum antibody against alloantigens cannot be detected using conventional techniques such as dot assays or that the immunological response to any alloantigens is not sufficient to interfere with the therapeutic effect of the transfused product.

The inventive method was carried out, in order to produce a preparation in accordance with the second aspect of the invention, as follows.

Freshly-collected, stored (one to five days at 20-25°C) or outdated (beyond five days at 4°C) platelet concentrates (50-60 ml/concentrate) were pooled in 600 ml blood bags (Fenwall transfer pack unit, 4R2023, Fenwall Laboratories, Deerfield, Illinois) via sterile plasma transfer sets (Fenwall 4C2243, Fenwall Laboratories, supra). Each bag contained a total of 500-600 ml of platelet concentrates (hereinafter, 600 ml unit). The 600 ml units were centrifuged at 1,000 rpm for 11 minutes at 22°C to remove contaminating red and white blood cells (PR7000, International Equipment Company, Needham Heights, Massachusetts). The supernatants, which contained the platelets, then were transferred to new 600 ml blood bags and centrifuged at 3,000 rpm for 25 minutes at 22°C to separate platelets from plasma. Platelet-poor plasma was expressed and each of the resulting platelet pellets was gently resuspended in 20 ml of an 0.9% NaCl solution (physiological saline), diluted to a final volume of 100 ml with additional saline, and then pooled in 300 ml blood bags (three 100 ml samples per bag corresponding to three original 600 ml units). The resuspended platelets again were pelleted by centrifugation at 3,000 rpm for 20 minutes at 22°C. The supernatant was removed and the platelet pellet was washed twice with physiological saline by repeated resuspension and centrifugation.

The washed platelets were finally resuspended in physiological saline (25 ml per each 600 ml unit) and disrupted by repeated freezing (at -80°C for at least six hours) and thawing (at 25°C for at least one hour), three times. The frozen and thawed suspension was diluted with physiological saline (100 ml per each 600 ml unit) and centrifuged at 3,000 rpm for 30 minutes to collect a platelet ghost pellet. This platelet ghost pellet was resuspended in physiological saline (100 ml per each 600 ml unit) and washed twice by repeated resuspension and centrifugation.

Other methods could have been employed to disrupt platelet membranes and to isolate such a platelet ghost fraction. For example, platelets may be equilibrated with glycerol and then may be broken hypotonically by rapid dilution of the glycerol concentration external to the cell. This creates an osmotic pressure gradient across the external membrane of the platelets, which leads to a rupture of the cell membrane. In addition, many other chemical agents (e.g., NaCl) may be used in a similar manner to induce osmotic shock and disrupt platelets.

The washed ghost pellet was resuspended in physiological saline (40 - 50 ml per each 600 ml unit) and heated at 60°C for 20 hours in a water bath. Alternatively, the platelet ghost suspension may be heated to 100°C for five minutes. These conditions are sufficient to inactivate any viral contaminants. A gross precipitate developed during the heat treatment. This heat-treated, platelet ghost suspension then was homogenized in a sonicator (ultrasonic processor Model W-385, Heat Systems, Inc., Farmingdale, New York) using a 1/2" disruptor horn in a flow cell (Model 800B, Heat Systems). The sonicator system was flushed first with nitrogen prior to injection of the platelet ghost suspension. The suspension was sonicated by pulsing at 20kHz for 5 minutes and 43 seconds (2 second cycle, 1.4 seconds on, 0.6 seconds off) with output control setting at "4" to produce double amplitude of 48 micrometers. The sonicated preparation next was centrifuged at 3,000 rpm for 30 minutes at 22°C to separate the precipitated material from the formed platelet membrane microparticles which remain in the supernatant. The supernatant was removed and stored in sealed containers at either 4°C, -20°C or -80°C under nitrogen, or stored lyophilized under nitrogen. Unless otherwise indicated, microparticles stored at 4°C were used in the following procedures.

The platelet microparticle fraction prepared as described above was free of active virus. It also was substantially free of platelet ghosts, with greater than 80% of the microparticles being less than 600 nanometers in diameter and greater than 95% less than 1,000 nanometers. The average diameter of the microparticles prepared from newly outdated platelets (within two weeks after outdating) for 7 different preparations was between 300 and 400 nanometers. The mean diameter for the 7 preparations was 341 nanometers.

The platelet microparticle fraction also was substantially free of serotonin, GPIIb/IIIa (a surface glycoprotein), purine nucleoside phosphorylase, coagulation factors V, VIII, IX and X, and thrombospondin (an alpha granule component). On the other hand, GPIb (another surface glycoprotein) was present and Beta-glucuronidase (a lyzosomal marker) also was detectable (about 25% as a percent of lysate). The absence of GPIIb/IIIa was surprising since it is believed that GPIIb/IIIa is necessary for hemostasis.

The composition of the platelet microparticle fraction was determined in two different sets of experiments for certain components and is presented in Table I:

**Table I**

| PERCENT OF COMBINED WEIGHT (W/W) | | | | |
|---|---|---|---|---|
| EXPT. | CARBOHYDRATE | PHOSPHOLIPID | PROTEIN | CHOLESTEROL |
| N = 4 | 3.3±0.14 | 30.±0.9 | 57.8±0.9 | 9(assumed) |
| N = 8 | 2.6±.3 | 30.1±2.5 | 53.2±1.7 | 9.9±.9 |

The procoagulant activity of the preferred platelet microparticle fraction prepared from newly outdated platelets was determined using the Russel's viper venom time (5) which is used to measure PF-3 activity. The Russel assay is a thrombin generation test, for which the end point may be determined by fibrinogen clotting. The specific activity was determined by comparison to a thrombin standard curve and may be expressed as units (U) of thrombin generated per mg of platelet protein or per mg of platelet phospholipid. The PF-3 specific activity per mg protein (U/mg) was determined in a first set of experiments to be 148.1±13.4 (n=7). In a second set of experiments it was 175±8 (n=8). The PF-3 specific activity per mg phospholipid (U/mg) in the first set of experiments was 291.3±40.0. In the second set of experiments it was 310±23. These specific activities far exceed those for fresh, intact whole platelets. It is believed that this is due to the release of procoagulant membrane fragments normally unavailable in intact platelets.

The specific activity was retained even after lyophilization, although it was necessary to add a protective material to the preparation to retain greater than about 60% of the specific activity (sucrose at 0.4 gm/dl, >90% activity retained.)

The composition of the phospholipid portion of the preparation was determined in two different sets of experiments and is shown in Table II:

**Table II**

| PERCENT OF COMBINED PHOSPHOLIPID (W/W) | | | | | |
|---|---|---|---|---|---|
| EXP. | PI | PS | PE | PC | SP |
| N=4 | 5.8±1.0 | 10.1±0.9 | 22.5±1.5 | 45.8±4.0 | 15.4±.7 |
| N=8 | 6.8±.5 | 12.2±.7 | 23.8±2.1 | 40.6±2.3 | 16.6±1.5 |
| PI: phosphatidylinositol PE: phosphatidylethanolamine SP: sphingomyelin PS: phosphatidylserine PC: phosphatidylcholine | | | | | |

Platelet microparticle fractions were tested for their procoagulant activity and for their ability to control bleeding in thrombocytopenic animals. The procoagulant activity of a microparticle fraction prepared from newly outdated platelets was found to be comparable to one prepared from fresh platelets. Fractions prepared from fresh and newly outdated platelets had comparable procoagulant activity to whole platelets. The transfusion of this fraction shortened bleeding time in all recipient animals.

The effect of heat treatment and the effect of sonication on procoagulant activity also was tested. The microparticle fraction prepared from newly outdated platelets was found to be relatively stable to heat treatment. It further was discovered that the procoagulant activity of this platelet microparticle fraction was diminished greatly when homogenization preceded heat treatment. These and other properties of the microparticle fractions of the invention are set forth more fully in the examples below.

The product prepared according to the above-identified procedure, as well as various intermediates, were tested in immunological dot assays for the presence of GPIIb/IIIa. GPIIb/IIIa was present in the intact platelet preparations, the platelet lysate, the supernatant from the platelet lysate, and the platelet membrane ghost fraction. There was no detectable GPIIb/IIIa in a heated platelet membrane ghost fraction or in the final product.

The final product and intermediates also were tested in immunological dot assays for the presence of HLA antigens (Class I). HLA antigens were present in the washed, intact platelet preparations, the platelet lysate, the supernatant from the platelet lysate, and the platelet membrane ghost fraction. HLA antigens were not detectable in a heated platelet membrane ghost fraction or in the final product.

The final product and various intermediates further were tested in immunological dot assays for the presence of GPIb. All samples tested, including those heat treated, showed the presence of GPIb.
FIG.1 is a graph showing a standard curve for thrombin clotting time;
FIG.2 is a graph showing the effect of heat treatment of PF-3 specific activity;
FIG.3 is a graph showing the platelet count and bleeding time in normal thrombocytic and thrombocytopenic rabbits;
FIG.4 is a graph showing the effect of transfused platelet membrane microparticles on the bleeding time in doxorubicin hydrochloride-induced thrombocytopenic animals;
FIG.5 is a graph showing the effect of transfused platelet membrane microparticles on antibody induced thrombocytopenic animals; and
FIG.6 is a graph showing the dose-dependant effect of transfused platelet membrane microparticles on the bleeding time and busulfan-induced, severely thrombocytopenic animals.

### Example 1

PF-3 (platelet factor-3) procoagulant activity was measured by the Russel viper venom time assay, which is a thrombin generation test. The end point of the test is determined visually by the clotting of fibrinogen present in a pooled human plasma sample.

A stock solution of CaCl₂ (0.025 M in imidazol buffer, pH 7.3) was maintained at 37°C. Pooled human plasma and the platelet membrane microparticle fractions in solution (25 ug/ml in saline) were stored at room temperature. Russel viper venom (RVV; 10 ug/ml in saline; Wellcome Diagnostics, Dartford, England) was kept on ice.

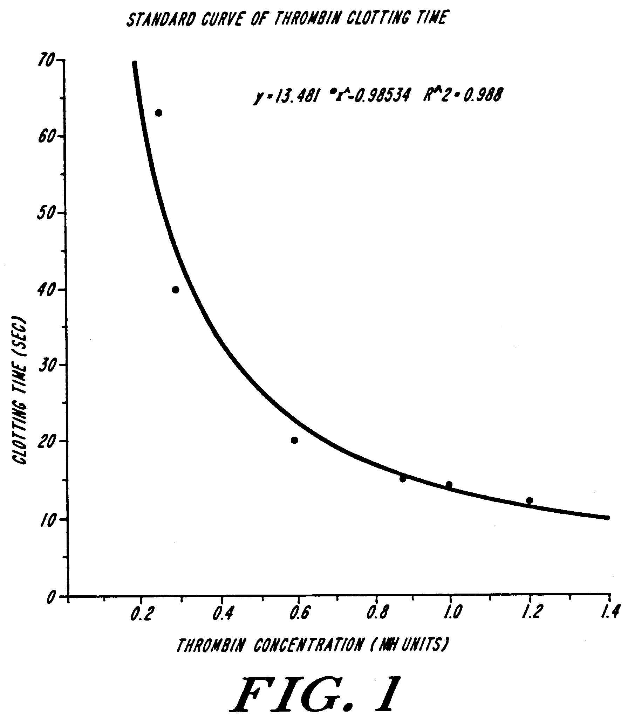

The assay was initiated by mixing and incubating 0.1 ml each of pooled plasma and the solution of platelet membrane microparticles in a borosilicate glass tube (12x75 mm) at 37°C for 5-10 minutes. RVV solution (0.1 ml) was then added and further incubated for 30 seconds at 37°C followed by adding 0.1 ml of CaCl₂ solution. The time between the addition of CaCl₂ solution and the detection of fibrin clotting was determined. The unit of thrombin generated by the assay system was calculated from a standard curve of thrombin clotting time (FIG. 1) using purified bovine thrombin (Sigma 850-1; Sigma Chemical Co., St. Louis, MO) and pooled human plasma.

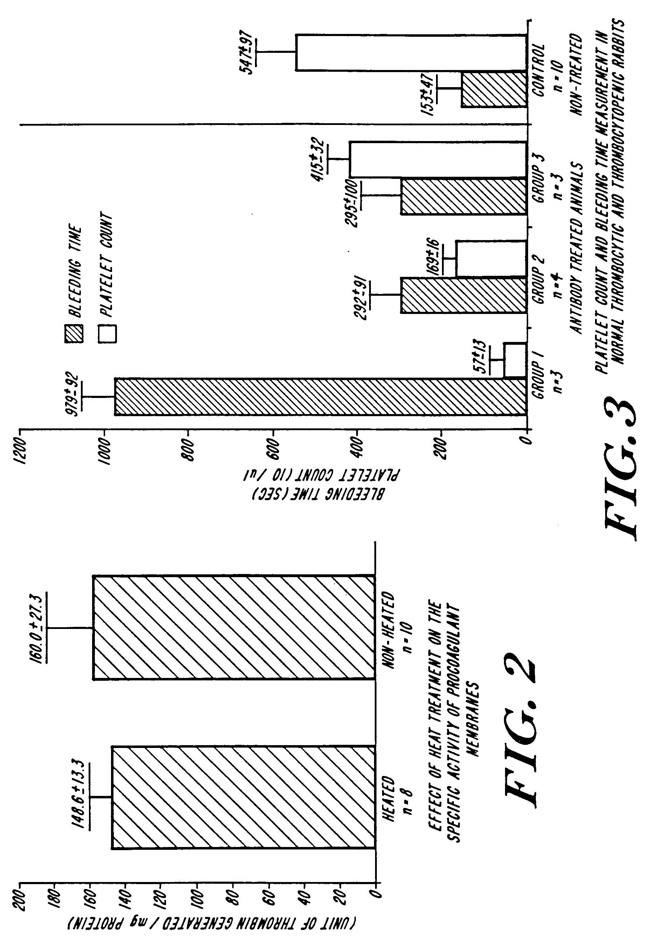

The effect of heat treatment and the effect of sonication on procoagulant activity (PF-3) using Russel's viper venom time (5) was tested. As illustrated in FIG. 2, heat treatment, followed by sonication, resulted in a platelet membrane microparticle fraction that retained a substantial amount of its procoagulant activity. However, when sonication preceded heat treatment (not shown), the procoagulant activity of the platelet membrane microparticle fraction was altered drastically, the procoagulant activity being reduced by 50%.

### Example 2

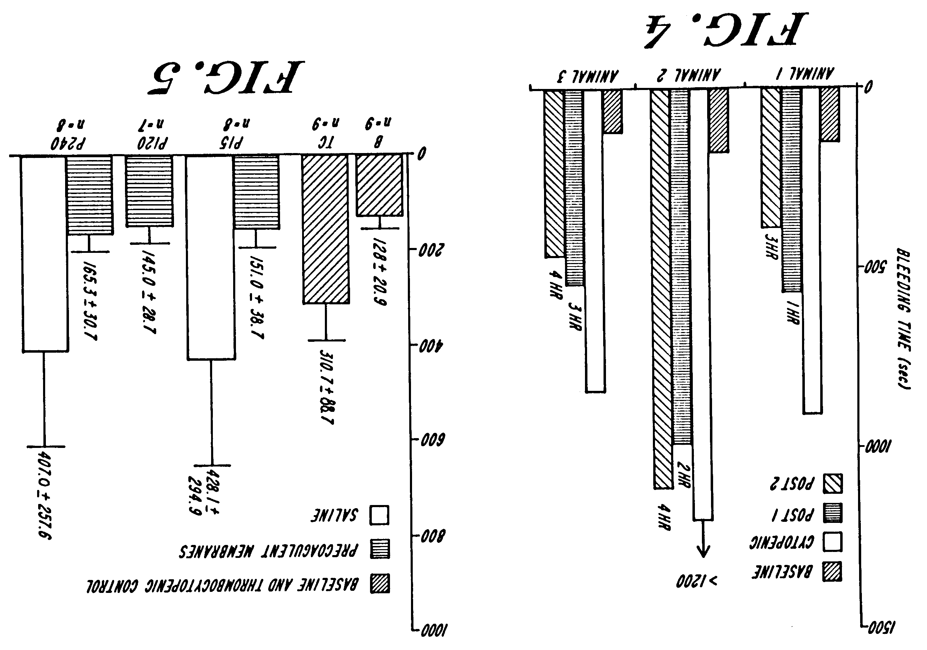

The platelet microparticles were tested for their ability to control bleeding in antibody-induced, thrombocytopenic animals. Anti-rabbit platelet antiserum was used to induce thrombocytopenia in rabbits. Platelet counts and bleeding time first were determined in 10 normal rabbits (body weight 3.53±0.41 Kg; platelet count, 548,000±97,000/ul; bleeding time, 153±47 sec; mean±SD). Anti-rabbit platelet antiserum was obtained from a commercial source then was administered intravenously to the 10 rabbits at a dose of 0.2-0.4 ml antiserum per kg body weight. At two hours after antiserum injection, thrombocytopenia of varying degree was present in all animals. Data were grouped according to the degree of "induced thrombocytopenia": GROUP 1, platelet count less than 80,000/ul; GROUP 2, platelet count between 100,000 - 200,000/ul and GROUP 3, platelet count above 200,000/ul (FIG. 3).

Prolongation of bleeding time also was induced in all 10 antiserum-treated rabbits (measured at two hours after antiserum injection). Marked prolongation occurred only in Group 1 animals (severe thrombocytopenia, <75,000/ul) and a moderate prolongation was observed in both Group 2 and 3 animals (FIG. 3).

Platelet microparticle fractions prepared from newly outdated Platelets were transfused (2 mg protein/kg body weight) into Group 1 animals with severe thrombocytopenia. Referring to FIG. 4, platelet counts obtained at two hours after antiserum injection were 41,000, 73,000 and 56,000/ul for animals 1, 2 and 3, respectively. At the same interval, bleeding time was marked prolonged in all three rabbits, especially rabbit number 2, in which profuse bleeding from the test site persisted even at 20 minutes after the initial incision and required application of local pressure to stop the bleeding. After receiving the platelet microparticles, animals 1 and 3 showed progressive shortening of bleeding time, while spontaneous arrest of bleeding occurred within 20 minutes in rabbit number 2, without the need of local pressure. Thus, a general trend of shortening of bleeding time after transfusion of the platelet microparticles of the invention was demonstrated in all recipient animals.

### Example 3

The platelet microparticles were tested for their ability to control bleeding in Doxorubicin hydrochloride-induced, thrombocytopenic animals. Doxorubicin hydrochloride (Adriamycin; 2mg/kg body weight) was administered intravenously to nine rabbits to induce thrombocytopenia. The baseline platelet count (before doxorubicin injection) was 488,000±94,000/ul. The baseline bleeding time was 128±21 sec. One week after doxorubicin injection, platelet count was reduced to 130,000±31,000/ul (p<0.01) with a 2.4 fold increase in bleeding time (311±89 sec; n=9) over the thrombocytopenic controls (p<0.01).

Platelet microparticle fractions (2 mg membrane protein/Kg body weight), prepared from newly outdated platelets or saline preparations were transfused into the thrombocytopenic rabbits induced by doxorubicin. Hemostatic efficacy was demonstrated in all animals that received procoagulant membranes. Repeat measurements of bleeding time at 15 minutes, two and four hours post-transfusion showed a significant shortening of bleeding time from thrombocytopenic controls (<0.01) (FIG. 5). In contrast, no significant differences were noted between measurements obtained before and after infusion of saline to thrombocytopenic rabbits.

### Example 4

The efficacy of the platelet microparticles of the invention in controlling bleeding was further confirmed by the dose-dependant response when the microparticles were administered to severely thrombocytopenic rabbits. Two injections of busulfan (totalling 35 mg/kg body weight) were administered subcutaneously to rabbits to produce severe thrombocytopenia (platelet count less than 25,000 per ul). Three different doses of platelet microparticles (0.5 mg/kg body weight, 1.0 mg/kg body weight and 2.0 mg/kg body weight) and a saline control preparation were transfused into the severely thrombocytopenic rabbits. Baseline platelet counts determined on the day of transfusion for all animals was on the order of about 15,000/ul.

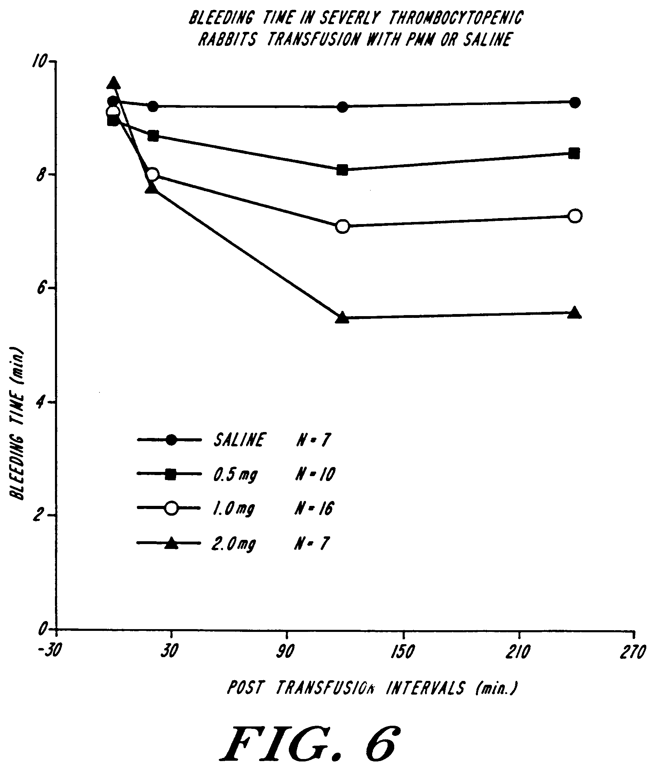

Referring to FIG. 6, the hemostatic efficacy of the platelet microparticles was directly related to the dose transfused. Transfusion of saline to the severely thrombocytopenic rabbits did not change the bleeding time. On the other hand, the prolonged bleeding time in severely thrombocytopenic rabbits was shortened in a dose-dependant manner when platelet microparticles were transfused.

The foregoing transfusion studies in thrombocytopenic rabbits demonstrate the hemostatic efficacy of the platelet membrane fragment preparation of the invention.

### References

1) Klein, E., Farber, S., Djersasi, I., Toch, R., Freeman, G., Arnold, P., "The Preparation and Clinical Administration of Lyophilized Platelet Material to Children with Acute Leukemia and Aplastic Anemia", J. Pediatrics, 49:517, 1956.
2) Hjort, P., Perman, V., and Cronkite, E., "Fresh, Disintegrated Platelets and Radiation Thrombocytopenia: Correction of Prothrombin Consumption without Correction of Bleeding".
3) McGill, M., Fugman, D., Vittorio, N., and Darrow, C., "Platelet Membrane Vesicles Reduced Microvascular Bleeding Times in Thrombocytopenic Rabbits", J. Lab. Clin. Med, 109:127-133, 1987.
4) Wu, V-Y., McCoy, L. E., "Platelet Factor 3: Quantitation and Characterization", Thromb. Res., 11:581-593, 1977.
5) Spaet, T.H., Cintron, J., "Studies on Platelet Factor-3 Availability", Brit. J. Hamematol, 11:269, 1965.
6) James N. George, Elaine B. Pickett, and Robert Heinz, "Platelet Membrane Microparticles in Blood Bank Fresh Frozen Plasma and Cyroprecipitate" Blood, Vol. 68, No. 1 (July), 1986; pp. 307-309

## Claims

1. A method for preparing a platelet derived product having procoagulant activity, characterised by heat-treating a preparation of isolated procoagulantly active platelets or platelet membrane fragments sufficiently thereby to inactivate any virus contained in said preparation.

2. A method as claimed in claim 1, wherein the product is platelet membrane derived and isolated from the preparation after heat-treatment.

3. A method as claimed in claim 1 or claim 2, wherein the heat-treatment renders said preparation non-immunogenic.

4. A method as claimed in any one of claims 1-3, wherein the heat-treatment renders said preparation non-antigenic with respect to class I HLA determinants.

5. A method as claimed in any one of claims 1-4, wherein the heat-treatment renders said preparation non-alloimmunogenic.

6. A method as claimed in any one of claims 1-5, wherein the heat-treatment eliminates substantially all GP IIb/IIIa complex from said preparation.

7. A method as claimed in any one of claims 1-6 further comprising homogenizing said preparation to form platelet membrane microparticles substantially free of ghost platelets.

8. A method as claimed in claim 7 wherein the preparation is sonicated after said heat-treatment, to form a fraction comprising the microparticles.

9. A method as claimed in claim 8 further comprising lyophilizing said preparation after sonication.

10. A method as claimed in any one of claims 1-7 comprising lyophilizing said platelet derived product.

11. A method as claimed in any one of claims 1-10, wherein said preparation comprises isolated outdated platelets or membrane fragments thereof.

12. A method as claimed in claim 11, wherein said preparation comprises pooled, outdated platelets or membrane fragments thereof.

13. A method as claimed in claim 8, further commprising separating any precipitate formed as a result of heating from said microparticle fraction.

14. A platelet membrane fragment preparation having procoagulant activity, characterised by comprising active virus free isolated platelet membrane fragments obtainable by using a method as claimed in at least one of claims 1-13.

15. A preparation as claimed in claim 14, which is non-immunogenic.

16. A preparation as claimed in claim 14 or 15, which is non-alloimmunogenic.

17. A preparation as claimed in any one of claims 14-16, which is nonantigenic with respect to class I HLA determinants.

18. A preparation as claimed in any one of claims 14-17 substantially free of GP IIb/IIIa complex.

19. A preparation as claimed in any one of claims 14-18, which is substantially free of ghost platelets.

20. A preparation as claimed in any one of claims 14-19, wherein said preparation is substantially free of vesicles having a diameter of greater that one µm.

21. A preparation as claimed in any one of claims 14-20, wherein said preparation is stable at 4°C for at least 8 weeks.

22. A preparation characterised by comprising a pharmaceutically effective amount of a preparation as claimed in any one of claims 14-20, in combination with a pharmaceutically acceptable carrier.

## Patentansprüche

1. Verfahren zur Herstellung eines von Blutplättchen abgeleiteten Produktes mit prokoagulierender Aktivität, gekennzeichnet durch ausreichende Wärmebehandlung einer Präparation von isolierten prokoagulierend wirksamen Blutplättchen oder Blutplättchenmembranfragmenten, um dadurch jegliches in der Präparation enthaltene Virus zu inaktivieren.

2. Verfahren, wie beansprucht in Anspruch 1, wobei es sich bei dem Produkt um Blutplättchenmembran handelt, welche nach Wärmebehandlung aus der Präparation abgeleitet und isoliert wird.

3. Verfahren, wie beansprucht in Anspruch 1 oder Anspruch 2, wobei die Wärmebehandlung die Präparation nicht-immunogen macht.

4. Verfahren, wie beansprucht in mindestens einem der Ansprüche 1 - 3, wobei die Wärmebehandlung die Präparation in Hinsicht auf Klasse I-HLA-Determinanten nicht-antigen macht.

5. Verfahren, wie beansprucht in mindestens einem der Ansprüche 1 - 4, wobei die Wärmebehandlung die Präparation nicht-alloimmunogen macht.

6. Verfahren, wie beansprucht in mindestens einem der Ansprüche 1 - 5, wobei die Wärmebehandlung im wesentlichen den gesamten GP IIb/IIIa-Komplex aus der Präparation eliminiert.

7. Verfahren, wie beansprucht in mindestens einem der Ansprüche 1 - 6, welches ferner das Homogenisieren der Präparation umfaßt, um Blutplättchenmembran-Mikropartikel zu bilden, welche im wesentlichen frei von Ghost-Blutplättchen sind.

8. Verfahren, wie beansprucht in Anspruch 7, wobei die Präparation nach der Wärmebehandlung ultraschallbehandelt wird, um eine die Mikropartikel umfassende Fraktion zu bilden.

9. Verfahren, wie beansprucht in Anspruch 8, das ferner das Lyophilisieren der Präparation nach der Ultraschall-Behandlung umfaßt.

10. Verfahren, wie beansprucht in mindestens einem der Ansprüche 1 - 7, welches das Lyophilisieren des aus Blutplättchen abgeleiteten Produktes umfaßt.

11. Verfahren, wie beansprucht in mindestens einem der Ansprüche 1 - 10, wobei die Präparation isolierte veraltete Blutplättchen oder Membranfragmente davon umfaßt.

12. Verfahren, wie beansprucht in Anspruch 11, wobei die Präparation vereinigte veraltete Blutplättchen oder Membranfragmente davon umfaßt.

13. Verfahren, wie beansprucht in Anspruch 8, welches ferner das Abtrennen jeglichen, als Ergebnis des Erwärmens aus der Mikropartikel-Fraktion gebildeten Präzipitates umfaßt.

14. Blutplättchenmembranfragment-Präparation mit prokoagulierender Aktivität, dadurch gekennzeichnet, daß sie aktive, virusfreie, isolierte Blutplättchenmembranfragmente umfaßt, die durch Anwenden eines Verfahrens, wie beansprucht in mindestens einem der Ansprüche 1 - 13, erhältlich sind.

15. Präparation, wie beansprucht in Anspruch 14, welche nicht-immunogen ist.

16. Präparation, wie beansprucht in Anspruch 14 oder 15, welche nicht-alloimmunogen ist.

17. Präparation, wie beansprucht in mindestens einem der Ansprüche 14 - 16, welche nicht-antigen in Hinsicht auf Klasse I-HLA-Determinanten ist.

18. Präparation, wie beansprucht in mindestens einem der Ansprüche 14 - 17, welche im wesentlichen frei von GP IIb/IIIa-Komplex ist.

19. Präparation, wie beansprucht in mindestens einem der Ansprüche 14 - 18, welche im wesentlichen frei von Ghost-Blutplättchen ist.

20. Präparation, wie beansprucht in mindestens einem der Ansprüche 14 - 19, wobei die Präparation im wesentlichen frei von Vesikeln mit einem Durchmesser von mehr als einem µm ist.

21. Präparation, wie beansprucht in mindestens einem der Ansprüche 14 - 20, wobei die Präparation bei 4°C mindestens 8 Wochen lang stabil ist.

22. Präparation, dadurch gekennzeichnet, daß sie eine pharmazeutisch wirksame Menge einer Präparation, wie beansprucht in mindestens einem der Ansprüche 14 - 20, in Kombination mit einem pharmazeutisch akzeptablen Träger umfaßt.

## Revendications

1. Méthode pour préparer un produit dérivé des plaquettes sanguines ayant une activité procoagulante, caractérisée par le traitement à chaud d'une préparation de plaquettes isolées procoagulantes, ou de fragments de membranes de plaquettes, suffisamment pour inactiver les virus contenus dans ladite préparation.

2. Méthode selon la revendication 1, dans laquelle le produit est dérivé de membranes de plaquettes et isolé de la préparation après ledit traitement à chaud.

3. Méthode selon l'une des revendications 1 ou 2, dans laquelle le traitement à chaud rend ladite préparation non-immunogène.

4. Méthode selon l'une quelconque des revendications 1 à 3, dans laquelle le traitement à chaud rend ladite préparation non-antigène par rapport aux déterminants de la classe I HLA.

5. Méthode selon l'une des revendications 1 à 4, dans laquelle le traitement à chaud rend ladite préparation non-alloimmunogène.

6. Méthode selon l'une des revendications 1 à 5, dans laquelle le traitement à chaud élimine sensiblement tous les complexes GP IIb/IIIa de ladite préparation.

7. Méthode selon l'une quelconque des revendications 1 à 6, comprenant au surplus une homogénéisation de ladite préparation de manière à former des microparticules de membranes de plaquettes pratiquement dépourvues de plaquettes fantômes.

8. Méthode selon la revendication 7, dans laquelle ladite préparation est soumise à des ultrasons après ledit traitement à chaud, de manière à former une fraction comprenant des microparticules.

9. Méthode selon la revendication 8 comprenant au surplus une lyophilisation de ladite préparation après qu'elle ait été soumise à des ultrasons.

10. Méthode selon l'une quelconque des revendications 1 à 7, comprenant la lyophilisation desdits produits dérivés des plaquettes.

11. Méthode selon l'une quelconque des revendications 1 à 6, dans laquelle ladite préparation comprend des plaquettes périmées isolées ou des fragments de membranes de celles-ci.

12. Méthode selon la revendication 11, dans laquelle ladite préparation comprend des plaquettes périmées regroupées ou des fragments de membrane de celles-ci.

13. Méthode selon la revendication 8, comprenant au surplus la séparation de tout précipité résultant du chauffage de ladite fraction à microparticules.

14. Préparation de fragments de membranes de plaquettes ayant une activité procoagulante, caractérisée en ce qu'elle comprend des fragments de membranes de plaquettes isolés libres de tout virus actif, que l'on peut obtenir en utilisant une méthode selon l'une quelconque des revendications 1 à 13.

15. Préparation selon la revendication 14, laquelle est non immunogène.

16. Préparation selon l'une des revendications 14 ou 15, laquelle est non-alloimmunogène.

17. Préparation selon l'une quelconque des revendications 14 à 16, laquelle est non-antigène par rapport au déterminant de classe I HLA.

18. Préparation selon l'une quelconque des revendications 14 à 17, pratiquement dépourvue de tout complexe GP IIb/IIIa.

19. Préparation selon l'une quelconque des revendications 14 à 18, qui est pratiquement dépourvue de plaquettes fantômes.

20. Préparation selon l'une quelconque des revendications 14 à 19, dans laquelle ladite préparation est pratiquement dépourvue de toute vésicule dotée d'un diamètre plus grand que un µm.

21. Préparation selon l'une quelconque des revendications 14 à 20, dans laquelle ladite préparation est stable à 4°C pour au moins 8 semaines.

22. Préparation caractérisée en ce qu'elle comprend une quantité de préparation pharmaceutiquement efficace selon l'une quelconque des revendications 14 à 20, en combinaison avec un support pharmaceutiquement acceptable.
